# EUROPEAN PATENT APPLICATION

(11) **EP 1 488 787 A1**
(43) Date of publication of application: **22.12.2004**
(21) Application number: 03013958.8
(22) Date of filing: 20.06.2003
(51) Int. Cl.: A61K 31/192, A61K 47/14, A61P 19/00, A61P 29/00

(54) **Topical stabilized formulations containing ketoprofen**

(71) Applicant: BERLIN-CHEMIE AG, 12489 Berlin (DE); Menarini Ricerche S.p.A., 00040 Pomezia (IT)
(72) Inventor: Baccani, Claudio, c/o Menarini Ricerche S.p.A, 00040 Pomezia (IT); Maggi, Carlo, Alberto c/o Menarini Ricerche S.p.A, 00040 Pomezia (IT); Manzini, Stefano, c/o Menarini Ricerche S.p.A, 00040 Pomezia (IT); Groeger, Karsten, c/o Berlin-Chemie AG, 12489 Berlin (DE); Schmiz, Reinhard, c/o Berlin-Chemie AG, 12489 Berlin (DE); Wihsmann, Marc, c/o Berlin-Chemie AG, 12489 Berlin (DE)
(74) Representative: Gervasi, Gemma, Dr.

(57) **Abstract**

The invention concerns topical pharmaceutical formulations containing ketoprofen or its S(+) isomer dexketoprofen together with an UV adsorber and an antioxidant.

## Description

### Field of the invention

The invention concerns topical pharmaceutical formulations containing ketoprofen or its S(+) isomer dexketoprofen together with an UV adsorber and an antioxidant. The formulations have, very low or no light-induced skin-irritating properties of any kind, not even when exposed to sun. In addition to that the pharmaceutical forms are well tolerated and show outstanding penetration rates.

### Prior art

Topical pharmaceutical forms including non-steroidal antirheumatics (NSAIDs), especially ketoprofen or dexketoprofen, are in the majority of reports generally described as skin-irritating, allergenic, phototoxic and photoallergenic , (Coz, Christophe J. et. al. (Contact Dermatitis, 38(5), 245ff (1989)) and Bosca, F. (J. Photochem.Photobiol., 43(3), 1ff (1998)).

Numerous investigations have been carried out in the past regarding the stabilisation against photochemical influences of formulations containing NSAID.

It has been tried to work non-steroidal antirheumatics (NSAIDs), especially ketoprofen or dexketoprofen, into ointments, creams or gels in such way that they remain largely stable, both physically and chemically.

Investigations of Le Coz, Christophe J. et al. and Bosca, F. could demonstrate that certain parts of the chemical structure of NSAID are photosensitively inert but others are not. In the compounds mentioned, benzophenone units are the active centres, while the propionic acid residue will not be affected by the interaction with light. The specified sensitivity is based on the property of the diaryl-keto-group to initiate oxidation processes, which means that activated triplet states in the molecule are made to stabilise themselves by a (intramolecular) redox reaction in a single-step reaction or also through a primary electron transfer with subsequent proton transfer.

Costanzo, L. L. et. al. have found out (Photochem. Photobiol., 50(3), 359ff. (1989)), that the four impurities [(3-benzoylphenyl)ethane, (3-benzoylphenyl)ethyl hydroperoxide, (3-benzoylphenyl)ethanol, and (3-Benzoylphenyl) ethanone] result from ketoprofene under aerobic conditions, while anaerobic exclusively 3-benzoylphenyl ethane is formed.

The recommendations of Klimstra, Paul Dale, et. al. (WO 9520387), to minimise toxic effects on the skin by timing the application of the active substance were successful in such cases only where the formulation was limited to the hours of the night.

JP 60155111 describes a pharmaceutical formulation for external use, containing ketoprofen, obtained by incorporating an ultraviolet light absorber, selected in the group of derivatives of p-aminobenzoic acid (PABA), anthranilic acid, benzophenone, salycilic acid or amino acid, and, if necessary, an antioxidant selected in the group ascorbic acid, stearic acid ester or tocopherol.

In FR 2804024 the authors describe an invention concerning topical pharmaceutical forms containing an non-steroidal antirheumatic, in particular ketoprofen, protected against light by sunscreens selected in the group: derivatives of cinnamic acid, dibenzoylmethane, benzophenone, p-amino benzoic acid. with the eventual presence of an antioxidant selected in the group: butylhydroxyanisol, t-butyl-p-cresol, palmytil ascorbate or tocopherol. The solutions described in the examples of FR2804024 were not found completely satisfactory, in particular in the case of ketoprofen.

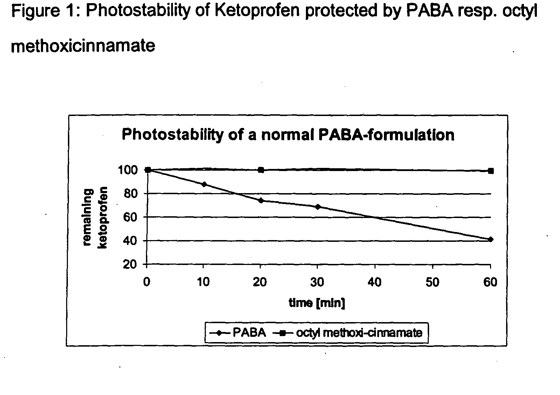

In fact examples 1 to 9: describe formulations containing ketoprofen with ethoxylate ester of p-amino benzoic acid (UV adsorber) and t-butyl hydroxyanisole (antioxidant), but we noticed that decomposition of ketoprofen was still present after irradiation (up to about 40 %; s. Figure 1) phototoxicity problems were found and permeation in human skill was reduced;
- in example 10 the use of octyl methoxycinnamate together with butyl hydroxyanisole did not prevent photodegradation of tenoxicam and problems with the permeation can be assumed caused the high amount of surfactant that had to be used;
a formulation similar to that of example 11 with sulisobenzone and butylhydroxyanisole, but using ketoprofen instead of diclofenac, was phototoxic and showed a reduced permeation in human skin.

Therefore formulations prepared according to what is claimed in JP60155111 and FR2804024 are not completely satisfactory. It can be said that in general they were found to be phototoxic. Furthermore it has to be noticed that the pharmacological properties were markedly changed when compared with known topical formulations containing ketoprofen without any UV adsorber (such as Ketum® ). On the one hand the described UV-blockers cause chemical resp. physical changes (coloration, decrease of viscosity, modified odour etc.) of the formulations. On the other hand important pharmacological properties, e.g. permeation rate, photo-allergy were influenced. Normally surfactants were used to stabilise the system or to dissolve the UV-blocker. In that case the permeation in human skin is reduced to a fractional part of the value achieved without UV-blocker, The photoallergic potential increased with the presence of the selected UV-light absorbers.

In the case that the UV-blocker itself has surface-active properties (e.g. ethoxylated para-aminobenzoic acid derivatives) a markedly decrease of the permeation rate is observed

In conclusion the described prior art is teaching to the man skilled in the art how to increase the photostability of topical formulation containing ketoprofen, but, so far, not to prevent phototoxic, photoallergic and permeation problems that still remains to be properly solved.

### Description of the figures

In figure 1 the percentage of degraded ketoprofen versus time was shown for two composition containing ketoprofen protected respectively by PABA (ethoxylated ester of p-amino benzoic acid ) and Eusolex 2292 (octyl methoxy cinnamate)

### Description of the invention

Surprisingly it was found that only systems consisting of ketoprofen or dexketoprofen, together with derivatives of cinnamic acid as the sunscreen, propyl gallate as an antioxidants, and other ecipients, e.g. flavours, penetration enhancers, antioxidants and additional solvents, show a prevention of phototoxic resp. photoallergenic properties.

In case of utilisation, as UV filter, of benzophenone-, antranilic acid-, salicylic acid-, amino acid based compounds or para-aminobenzoic derivatives, as taught by JP60155111 or FR2804024, a photo-protection, (frequently not complete), of the active ingredient was observed but the system itself was nevertheless phototoxic and the pharmacological and physical properties were significantly changed; even the addition of an antioxidant according to the teaching of JP60155111 did not solve the above mentioned problems. In case of utilisation of cinnamic acid derivatives without propyl gallate it also was observed that the active ingredient was photo-protected but nevertheless phototoxicity was detected in many cases. Other antioxidants (e.g. α-tocopherole, bytyl-hydroxyanisole, ascorbic acid) were investigated but they did not show results comparable to propyl gallate.

In addition problems of the formulations containing ketoprofen/dexketoprofen concerning the permeation of the active ingredient in human skin could be solved.

The invention was therefore based on the task to develop a product with a high percentage of ketoprofen or dexketoprofen, but noticeably lower phototoxic and photoallergenic effects, a high permeation rate in human skin and with preservation of the pharmacological qualities compared with formulations without sunscreen.

According to the invention, the topical pharmaceutical formulation is characterised by
- 0.5 to 5 % by weight of ketoprofen or dexketoprofen,
- 0.5 to 7 % by weight of derivatives of cinnamic acid selected in the group.of cinnamates
- 0.01-1,5% by weight of an ester of gallic acid, preferably propyl gallate
- and a residual content of up to 100 % by weight of pharmaceutical acceptable excipients, adjuvants, carriers, enhancers.

For the purposes of the present invention, the following specification have to be considered:
Benzophenones compounds known under the INCI names benzohenone-1 to benzophenone-11, e. g. benzophenone-3 (2-hydroxy-4-methoxybenzophenone) or benzophenone-4 (2-hydroxy-4-methoxybenzophenone-5-sulphonic acid).
Derivatives of cinnamic acid: esters of cinnamic acid selected in the group: methyl, ethyl, propyl, isopropyl, butyl, isobutyl, amyl, isoamyl, hexyl, heptyl, n-octyl, 2-ethylhexyl, eventually substituted on the aromatic ring by one or two groups selected among : hydroxy, metoxy, ethoxy
Derivatives of para-aminobenzoic acid, compounds as known under the INCI name 4-Bis(polyethoxy)para-aminobenzoic acid polyethoxyethyl ester.
Esters of gallic acid: esters selected in the group methyl, ethyl, propyl, isopropyl, butyl, amyl, isoamyl

As the result only derivatives of cinnamic acid in combination with propyl gallate are suitable. Formulations with derivatives of cinnamic acid with propyl gallate are higly preferred.

According to this invention the active substance of the pharmaceutical topical formulation is a compound selected among ketoprofen, the S(+) isomer dexketoprofen or a mixture of the two isomers of ketoprofen; these can be considered in the form of free acid or as one of their pharmaceutically acceptable salts selected in the group of salts of sodium, potassium, calcium, magnesium, tromethamine, hydroxyethylamine, dihydroxyethylamine, trihydroxyethylamine, lysine, arginine.

The preferred content of active substance of the non-steroidal antirheumatic is between 2 and 5 per cent by weight, particularly in the range from 2.5 to 5 per cent by weight, related to the overall composition. Special priority have the ranges between 2 and 4 per cent by weight and especially 2.5 to 4 per cent by weight.

The percentage of the ultraviolet filter is preferably between 0.5 and 5 per cent by weight, referred to the overall composition, particularly the range from 1 to 4 per cent by weight.

The percentage of the antioxidants is preferably between 0.01 and 1 per cent by weight, referred to the overall composition, particularly the range from 0.01 to 0.1 per cent by weight.

The formulations of this invention may contain further carriers/enhancers and adjuvants such as water, monovalent alcohols such as ethanol, vitamins, colorants, pigments with colouring effect, radical traps, thickeners, plasticizers, moisturisers, scents such as lavender oil, polyoles, esters, electrolytes, gel forming agents, polar and non-polar oils, polymers, copolymers, emulsifiers, emulsion stabilisers.

Even permeation enhancers and also usual liposomes can be used to transport the active substance or the active substance mix within the preparation according to this invention.

Further adjuvants or active substances in cosmetic formulations could be vitamins, e. g. vitamin A or vitamin-A derivatives; vitamin E or vitamin-E derivatives; complex vitamins, coloured or uncoloured plant extracts.

The preparation invented can be applied as creams, lotions, powders, sprays, aerosols and gels. In gel production, suitable gel forming agents can be used. These include carbomers, e. g. carbomer 940 (USP 24-NF19; p. 2427 (2000)), xanthane gum, carrageenan, acacia gum, guar gum, agar gel, alginates and methylhydroxyethylcellulose, carboxymethylcellulose, hydroxypropylcellulose, methylhydroxypropylcellulose, hydroxyethylcellulose, certain polyacrylates, polyvinyl alcohol, polyvinylpyrrolidone, montmorillonite etc.

Other active substances which can be used in the preparation as per invention are moisturisers such as urea or panthenol, vitamins, enzymes, vegetable active substances, polymers, melanin and natural antiphologistic active substances.

The preferred preparations are so characterised:
- 0.5-5 % of ketoprofen or S(+)ketoprofen or a mixture of the isomers
- 1-5 % of an ultraviolet filter (cinnamate derivatives)
- 0-2 % or preserving agents
- 0-20 % of permeation enhancers
- % of propyl gallate
- % scents
- 20-90 % of ethanol
- water

The preserving agents can be the standard substances used in pharmacy and cosmetics, such as esters of the hydroxybenzoic acid, parabenes, butylhydroxyanisole.

As permeation enhancers, e. g. urea, pyrrolidone, N-methylpyrrolidone, decylmethylsulfoxid, sodium-laurylsulfate and dimethylsulfoxide have been applied with success.

The preparation as invented shows - besides the antirheumatic effect - a noticeably lower photoallergenic effect or even no photoallergenic effect at all.

That seems to be particularly essential as S. Baudot et al. (Therapie, 53, 137ff. (1998)) have found out that the irritating, allergenic, phototoxic and photoallergenic properties of the NSAID formulations require hospitalisation of the patients in 10 % of all cases.

That can almost be excluded for the application of the preparation as invented.

It has also been quite surprising that the combination of an ultraviolet filter, the octylmethoxy cinnamate with an antioxidant, propyl gallate, and ketoprofen or dexketoprofen can exclude completely or almost completely an otherwise usual light-induced allergenic activity.

Thus, the inventions also concerns the use of a combination of an ultraviolet filter, octylmethoxy cinnamate, and an antioxidant, selected from the gallates for the manufacture of topical non-steroidal antirheumatics without photoallergenic or other light-induced side effects or with noticeably reduced side effect.

In the following, the invention shall be explained in detail by examples. All data are specified in per cent by weight, unless indicated otherwise.

### Example 1

A preparation was made bv mixing the individual inqredients in the normal wav

| | |
|---|---|
| Ketoprofen | 2.5 g |
| Hydroxypropylmethylcellulose | 2.1 g |
| Ethanol | 52.0 g |
| Lavender oil | 0.1 g |
| Octyl methoxycinnamate | 3.1 g |
| Diethyleneglycol-monoehtylether | 13.0 g |
| Propyl gallate | 0.05 g |
| Water | q. s. ad 100 g |

### Manufacturing description:

- the water is temperated at 40°C and with stirring the hydroxipropylmethylcellulose is quickly added. The mixture has -under temporarily stirring- to be swollen for 30min at 40°C in a closed vessel. After that time ketoprofen, lavender oil, octyl methoxycinnamate and propyl gallate were added to the aqueous gel. The mixture is slowly stirred until the temperature is below 30°C. Now diethyleneglycol-monoehtylether and ethanol were added and stirred in to reach a dispersion of the aqueous phase in the solvent. Under cooling the mixture is to be homogenised for 60 min.

### Example 2

| | |
|---|---|
| Ketoprofen | 3.8 g |
| Hydroxypropylcellulose | 2.1 g |
| Ethanol | 65 g |
| Lavender oil | 0.2 g |
| Octyl methoxycinnamate | 2.0 g |
| Diethyleneglycol-monoehtylether | 10.0 g |
| EDTA-Sodium | 0.1 g |
| Propyl gallate | 0.08 g |
| Water | q. s. ad 100 g |

### Manufacturing description:

The formulation was prepared according to the procedure of example 1.

### Example 3

| | |
|---|---|
| Dexketoprofen | 1.0 g |
| Hydroxypropylethylcellulose | 2.0 g |
| Ethanol | 55.0 g |
| Glycerine | 3.0 g |
| Lavender oil | 0.11 g |
| Octyl methoxycinnamate | 2.0 g |
| Diethyleneglycol-monoehtylether | 10.0 g |
| Propyl gallate | 0.02 g |
| Water | q. s. ad 100 g |

### Manufacturing description:

The formulation was prepared according to the procedure of example 1.

### Example 4

| | |
|---|---|
| Ethanol | 64.14 g |
| Glycerine | 3.0 g |
| Ketoprofen | 2.5 g |
| Octyl-methoxycinnamate | 3.0 g |
| Lavender oil | 0.2 g |
| Hydroxypropylmethylcellulose | 2.0 g |
| Propyl gallate | 0.06 g |
| Water | q. s. ad 100 g |

### Manufacturing description:

The water is temperated at 40°C and with stirring the hydroxipropylmethylcellulose is quickly added. The mixture has -under temporarily stirring- to be swollen for 30min at 40°C in a closed vessel.

In a second vessel ketoprofen, lavender oil, octyl methoxycinnamate, glycerine and propyl gallate were dissolved in ethanol.

The aqueous gel is slowly stirred until the temperature is below 30°C. Now the ethanolic solution was added to the gel and stirred in to reach a dispersion of the aqueous phase in the solvent. Under cooling the mixture is to be homogenised for 60 min.

### Example 5

| | |
|---|---|
| Ethanol | 54.5 g |
| Diethyleneglycol-monoehtylether | 10 g |
| Glycerine | 3 g |
| Ketoprofen | 2.5 g |
| Octyl methoxycinnamate | 2 g |
| Lavender oil | 0.2 g |
| Propyl gallate | 0.05 g |
| Carbomer 940 (USP) | 1.0 g |
| Hydroxypropylcellulose | 1 g |
| Urea | 3 g |
| Water | q. s. ad 100 g |

### Manufacturing description:

The formulation was prepared according to the procedure of example with the difference that the mixture of hydroxypropylcellulose and carbomer was swollen in the aqueous solution of urea and ketoprofen, lavender oil, octyl methoxycinnamate, glycerine and propyl gallate were dissolved in ethanol/diethyleneglycol-monoehtylether.

### Example 6

| | |
|---|---|
| Ethanol | 45.0 g |
| Ketoprofen | 2.5 g |
| Octyl methoxycinnamate | 3.0 g |
| Lavender oil | 0.2 g |
| 2-Propanol | 20.0 g |
| Hydroxymethylpropylcellulose | 1.0 g |
| Hydroxypropylcellulose | 1.0 g |
| EDTA-Sodium | 0.1 g |
| Propyl gallate | 0.05 g |
| Trometamol | 0.1 g |
| Water q. s. ad | 100 g |

### Manufacturing description:

The formulation was prepared according to the procedure of example 5.

### Example 7

| | |
|---|---|
| Ethanol | 89.0 g |
| Ketoprofen | 2.5 g |
| Octyl methoxycinnamate | 1.0 g |
| Lavender oil | 0.2 g |
| Hydroxypropylcellulose | 2.0 g |
| Propyl gallate | 0.05 g |
| Bidist. Water | q. s. ad 100 g |

### Manufacturing description:

The formulation was prepared according to the procedure of example 1.

### Example 8

| | |
|---|---|
| Ethanol | 91.2 g |
| Ketoprofen | 2.5 g |
| Octyl methoxycinnamate | 1.0 g |
| Lavender oil | 0.2 g |
| Propyl gallate | 0.05 g |
| Hydroxypropylcellulose | 2.0 g |
| Pyrrolidone | 3.0 g |

### Manufacturing description:

Ethanol was filled in the reaction vessel and ketoprofen, octylmethoxycinnamate, lavender oil, propyl-gallate and pyrrolidone were dissolved. After reaching a clear solution (about 30min) hydroxypropylcellulose was added and vigorously stirred for 2 hours.

### Pharmacilogical tests

### Photodegradation

After irradiation with a xenon lamp Schott WG320/1.5 mm with 100 KLux h and 75 W h/m², percentage og degraded ketoprofen was determined: In Fig 1 the percentage of not-degraded ketoprofen is shown in function of time. With a similar procedure degradation of other compositions containing ketoprofen/UV filter/antioxidant agent prepared as described in FR2804024 and JP60155111 was determined (table 1).

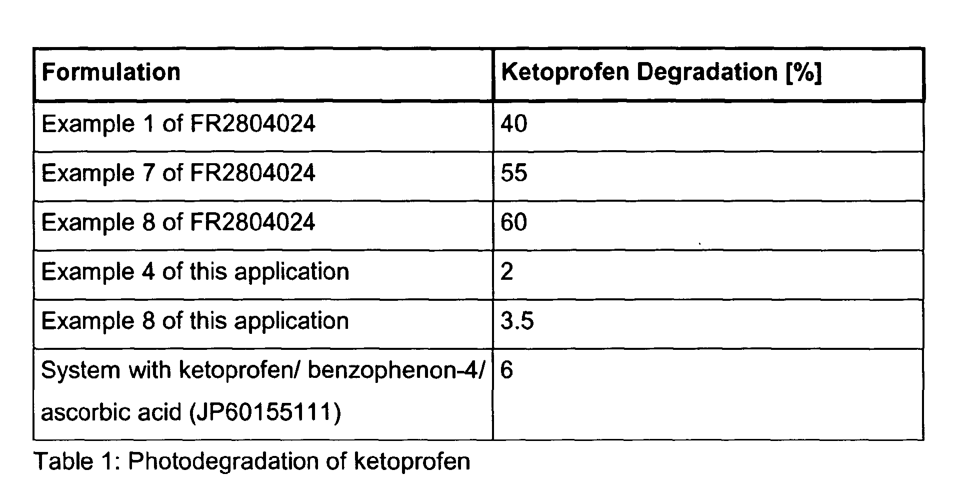

### Phototoxicity

A commercial sunscreen preparation with sunscreen factor 8 containing an ultraviolet filter based on inorganic filters (TiO₂/ZnO was applied to the skin on the back of two groups of 10 subjects each.

The product of the 1^{st} Example was applied to a group of 10 subjects (test group I) on the skin of the back (skin type II) as described in a), the amount being about 2 mg/cm². On the skin of a second group of 10 subjects (test group II, also skin type II) which was protected according to a), a product was applied which contained all constituents of the product of the 1^{st} Example, but no octyl methoxycinnamate. Radiation with a xenon lamp Schott WG320/1.5 mm with 100 KLux h and 75 W h/m² in 5 doses. The skin of the subjects was inspected visually after 24 hours.

No reddening of the skin was found in group I after 24 hours. In group II, the skin of 3 subjects had not reddened, 4 subjects showed a slight reddening of the skin, and 3 subjects a medium reddening of the skin after 24 hours.

### Photoallergenic reaction

Several compositions containing Ketoprofen, a sunscreen and a permeation enhancer a.o. were compared with the corresponding placebos. All of the compositions were investigated concerning their in vitro phototoxicity (neutral red uptake test). The test was performed according to the COLIPA standard operating procedure. As the result it could be shown that the placebos are not phototoxic.

For the formulations containing active ingredient, an antioxidants and octyl methoxycinnamate in a sufficient amount no phototoxicity was observed. In case of the utilisation of other sunscreens the formulations were phototoxic although the active ingredient was stabile during the irradiation.

From these results the conclusion may be drawn that the NSAID/cinnamate/propylgallat combination does not reveal a photoallergenic reaction.

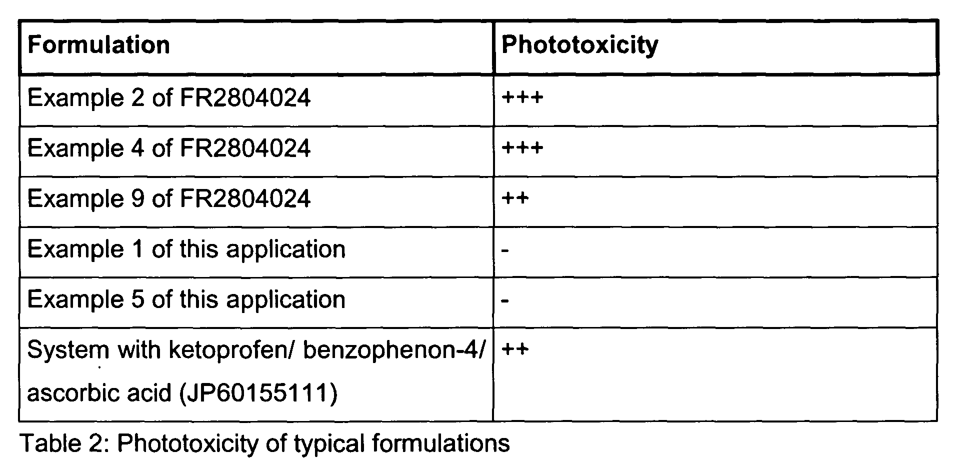

### Permeability

In Table 3 results of permeability throught human skin are shown for several compositions, all in comparison with to the corresponding formulation without sunscreen and antioxidant agent.

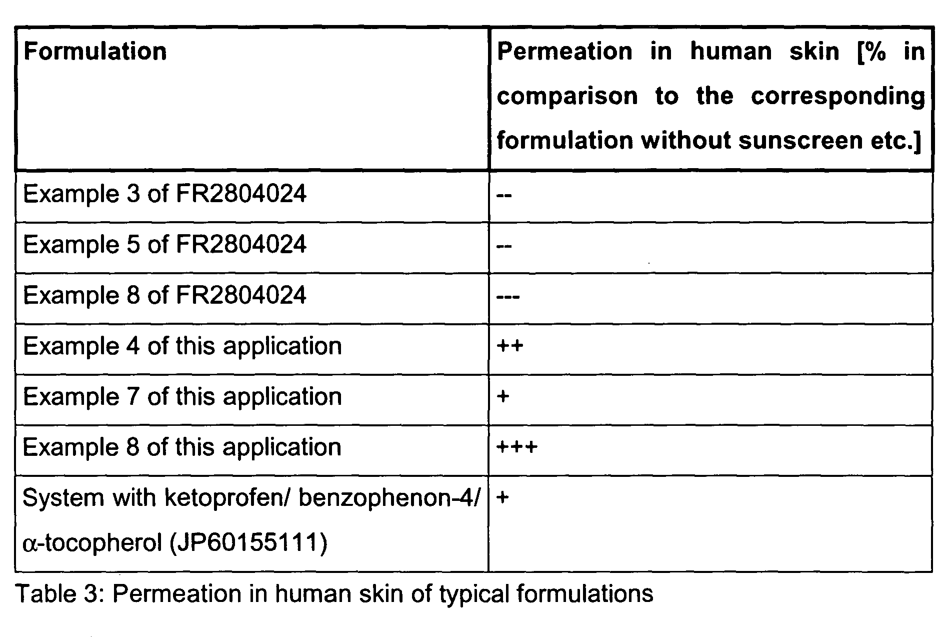

## Claims

1. Pharmaceutical topical composition containing:
a) a non-steroidal antiinflammatoty agent selected among: ketoprofen, the S(+) isomer dexketoprofen or a mixture of the two isomers of ketoprofen; these can be considered in the form of free acid or as one of their pharmaceutically acceptable salts selected in the group of salts of sodium, potassium, calcium, magnesium, tromethamine, hydroxyethylamine, dihydroxyethylamine, trihydroxyethylamine, lysine, arginine, or as mixtures of free acid with one of its salts selected in the above group;
b) an ester of cinnamic acid, acting as an UV filter, selected in the group of esters: methyl-, ethyl-, propyl-, isopropyl-, butyl-, isobutyl-, amyl-, isoamyl-, hexyl-, heptyl-, n-octyl-, 2-ethylhexyl-ester, eventually substituted on the aromatic ring by one or two groups selected among : hydroxy, metoxy, ethoxy;
c) an ester of gallic acid, acting as antioxidant agent, selected in the group of: methyl-, ethyl- propyl-, isopropyl-, butyl-, amyl-, isoamyl-ester.

2. Pharmaceutical topical composition according to claim 1, **characterised in that** the UV filter is 2-ethyl-hexyl p-methoxycinnamate.

3. Pharmaceutical topical composition according to claims 1 and 2, wherein the antioxidant agent is propyl gallate.

4. Pharmaceutical topical composition according to claims 1-3, wherein ketoprofen concentration, as free acid, is in the range 1-5% by weight when in racemic form or in the range of 0.5-2.5% by weight when the S(+) isomer is considered.

5. Pharmaceutical topical composition according to claim 4, wherein ketoprofen concentration , as free acid, is in the range 2.5-5% by weight when in racemic form or in the range of 1-2% by weight when the S(+) isomer is considered.

6. Pharmaceutical topical composition according to claim 5, wherein ketoprofen concentration , as free acid, is in the range 2.5-4% by weight when in racemic form.

7. Pharmaceutical topical composition according to claim 2, wherein the UV filter octyl methoxy cinnamate is in the range 0.5-5% by weight.

8. Pharmaceutical topical composition according to claim 7, wherein the UV filter octyl methoxy cinnamate is in the range 1-4% by weight.

9. Pharmaceutical topical composition according to claim 3, wherein the antioxidant agent propyl gallate is in the range 0.01-1% by weight.

10. Pharmaceutical topical composition according to claim 9, wherein the antioxidant agent propyl gallate is in the range 0.01-0.1% by weight.

11. Pharmaceutical topical composition according to claims 1 - 10 in the form of cream, gel, spray, lotion, or powder wherein the antiinflammatory agents is protected by photodegradation and the composition has no residual phototoxic and photoallergenic effects after application on the skin.

12. Use of a derivative of cinnamic acid selected in the group of esters: methyl, ethyl, propyl, isopropyl, butyl, isobutyl, amyl, isoamyl, hexyl, heptyl, n-octyl, 2-ethylhexyl, eventually substituted on the aromatic ring by one or two groups selected among : hydroxy, methoxy, ethoxy as UV filter in the preparation of a pharmaceutical composition according to claim 1, for the topical treatment of inflammatory pathology.

13. Use of propyl gallate, as antioxidant agent, in the preparation of a pharmaceutical composition according to claim 1, for the topical treatment of inflammatory pathology.
